(19) Europäisches Patentamt
European Patent Office
Office européen des brevets

(11) EP 0 787 000 B1

(12) **EUROPEAN PATENT SPECIFICATION**

(45) Date of publication and mention of the grant of the patent:
**08.11.2000 Bulletin 2000/45**

(21) Application number: **95933731.2**

(22) Date of filing: **29.08.1995**

(51) Int Cl.[7]: **A61K 31/505**

(86) International application number:
**PCT/US95/11186**

(87) International publication number:
**WO 96/06616 (07.03.1996 Gazette 1996/11)**

(54) **QUINAZOLINONE PHARMACEUTICALS AND USE THEREOF**

CHINAZOLINON-ARZNEIMITTEL SOWIE DEREN VERWENDUNG

COMPOSITIONS PHARMACEUTIQUES A BASE DE QUINAZOLINONE ET LEUR UTILISATION

(84) Designated Contracting States:
**AT BE CH DE DK ES FR GB GR IE IT LI LU MC NL PT SE**

(30) Priority: **31.08.1994 IL 11083194**

(43) Date of publication of application:
**06.08.1997 Bulletin 1997/32**

(73) Proprietors:
- **AGRICULTURAL RESEARCH ORGANIZATION, MINISTRY OF AGRICULTURE, STATE OF ISRAEL**
  **50250 Bet Dagan (IL)**
- **HADASIT MEDICAL RESEARCH SERVICES & DEVELOPMENT COMPANY, LTD.**
  **91120 Jerusalem (IL)**

(72) Inventors:
- **NAGLER, Arnon**
  **Jerusalem (IL)**
- **SLAVIN, Shimon**
  **Jerusalem (IL)**
- **VLODAVSKY, Israel**
  **90805 Mevaseret Zion (IL)**
- **PINES, Mark**
  **Rehovot (IL)**

(74) Representative: **Goodanew, Martin Eric et al**
**MATHISEN, MACARA & CO.**
**The Coach House**
**6-8 Swakeleys Road**
**Ickenham Uxbridge UB10 8BZ (GB)**

(56) References cited:
**US-A- 3 320 124** **US-A- 4 340 596**
**US-A- 5 449 678**

- **BIOCHIMICA ET BIOPHYSICA ACTA, Volume 1156, issued 1993, GRANOT et al., "Halofuginone: An Inhibitor of Collagen Type I Synthesis", pages 107-112.**
- **CIRCULATION RESEARCH, Volume 68, Number 1, issued January 1991, LINDNER et al., "Role of Basic Libroblast Growth Factor in Vascular Lesion Formation", pages 106-113.**
- **ARCHIVES OF SURGERY, Volume 130, Number 3, issued March 1995, CHIO et al., "Halofuginone, a Specific Collagen Type I Inhibitor, Reduces Anastomotic Intimal Hyperplasia", pages 257-261.**
- **POULTRY SCIENCE, Volume 70, Number 7, issued July 1991, GRANOT et al., "Increased Skin Tearing in Broilers and Reduced Collagen Synthesis in Skin in Vivo and in Vitro in Response to the Coccidiostat Halofuginone", pages 1559-63.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 787 000 B1

## Description

The present invention relates to compositions containing quinazolinones. More particularly, the present invention relates to a composition for the inhibition of restenosis, comprising a quinazolinone derivative as herein defined as active ingredient therein.

In U.S. Patent 3,320,124, issued in 1967, there is described and claimed a method for treating coccidiosis with quinazolinone derivatives.

Halofuginone, otherwise known as 7-bromo-6-chloro-3-[3-(3-hydroxy-2-piperidinyl)-2-oxopropyl]-4(3H)-quinazolinone, was first described and claimed in said patent by American Cyanamid Company, and was the preferred compound taught by said patent and the one commercialized from among the derivatives described and claimed therein.

Subsequently, U.S. Reissue Patent 26,833 and U.S. Patents 4,824,847; 4,855,299; 4,861,758 and 5,215,993 all relate to the coccidiocidal properties of halofuginone, while U.S. Patent 4,340,596 teaches that it can also be used for combatting theileriosis. Poultry Science, 70, 1559-1563 (1991) teaches that halofuginone causes an increase in skin tearing in poultry. Biochimica et Biophysica Acta, 1156, 107-112 (1993) reports effects of halofuginone on collagen metabolism in cell culture.

In recently filed US Patent Application Serial No. 08/181,066, now issued as US Patent No. 5449678, there is described and claimed an anti-fibrotic composition, comprising an amount of a compound of formula I

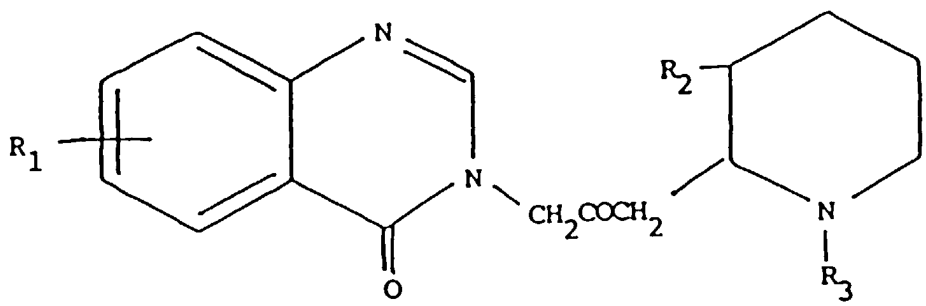

(I)

wherein:

- $R_1$ is a member of the group consisting of hydrogen, halogen, nitro, benzo, lower alkyl, phenyl and lower alkoxy;
- $R_2$ is a member of the group consisting of hydroxy, acetoxy, and lower alkoxy, and
- $R_3$ is a member of the group consisting of hydrogen and lower alkenoxy-carbonyl;

effective to inhibit collagen type I synthesis, as active ingredient therein.

After further research and development, it has now been discovered that the above-identified compounds of formula I are effective in the inhibition of restenosis, which formally is not a fibrotic condition.

The pathogenesis of atherosclerosis involves abnormal migration and proliferation of smooth muscle cells (SMCs) infiltrated with macrophages and embedded in extracellular matrix (ECM) of adhesive glycoproteins, proteoglycans and collagens [V. Fuster, et al., "The Pathogenesis of Coronary Artery Disease and the Acute Coronary Syndromes," New Eng. J. Med., Vol. 336, pp. 242-250 (1992); R. Ross, "The Pathogenesis of Atherosclerosis: A Perspective for the 1990's," Nature, Vol. 362, pp. 801-809 (1993)]. Under physiological conditions, the majority of arterial SMCs remains in the Go phase and cell growth is controlled by a balance between endogenous proliferation-stimulating and proliferation-inhibiting factors. Following endothelial cell perturbation due to atherogenic risk factors (i.e., hypertension, hyperlipoproteinemia, diabetes mellitus), platelets and non-platelet-derived growth factors and cytokines are released and stimulate monocyte and SMC migration as well as SMC proliferation (V. Fuster, et al., ibid.; R. Ross, ibid.). Among these growth factors are platelet-derived growth factor (PDGF) [G.A.A. Ferns, et al., "Inhibition of Neoinitmal Smooth Muscle Accumulation after Angioplasty by an Antibody to PDGF," Science, Vol. 253, pp. 1129-1132 (1991)], basic fibroblast growth factor (bFGF) [V. Lindner, et al., "Role of Basic Fibroblast Growth Factor in Vascular Lesion Formation," Circ. Res., Vol. 68, pp. 106-113 (1991)], and interleukin-1 (IL-1) [H. Loppnow and P. Libby, "Proliferating or Interleukin-1 Activated Human Vascular Smooth Muscle Cells Secrete Copious Interleukin 6," J. Clin. Invest., Vol. 85, pp. 731-738 (1990)]. Macrophages and platelets also release enzymes, i.e., elastase, collagenase, heparanase) that digest various constituents of the ECM and release bFGF and possibly other growth factors (TGFB) that are stored in basement membranes and ECM [I. Vlodavsky, et al., "Extracellylar Matrix-bound Growth Factors, Enzymes and Plasma Proteins," in: Molecular and Cellular Aspects of Basement Membranes, Monographs in Cell Biology, D.H. Rohrbach and R. Timpl, Eds., Academic Press, New York, New York, U.S.A., pp. 327-346 (1993)]. A

potent growth-promoting activity towards SMCs is also exerted by thrombin, which, under certain conditions, may be present within the vessel wall [R. Bar-Shavit, et al., "Thrombin Immobilized to Extracellular Matrix Is a Mitogen for Vascular Smooth Muscle Cells: Non-Enzymatic Mode of Action," Cell Reg., Vol. 1, pp. 453-463 (1990); S.M. Schwartz, "Serum-Derived Growth Factor is Thrombin?" J. Clin. Invest., Vol 91, p. 4 (1993)]. Molecules that interfere with the growth-promoting activity of these growth factors may attenuate the progression of the atherogenic process.

Proliferation of arterial smooth muscle cells (SMC) in response to endothelial injury is a basic event in the process of restenosis of coronary arteries after percutaneous transluminal coronary angioplasty (PCTA) [V. Fuster, et al., ibid.]. Coronary bypass surgery or angioplasty are applied to reopen coronary arteries that have been narrowed by heart disease. A major problem with both procedures is that arteries rapidly reclog in about 30% of patients undergoing angioplasty and about 10% of bypass surgery patients. Vascular SMC are ordinarily protected by the smooth inner lining of the arteries, composed of vascular endothelial cells. However, following bypass surgery or angioplasty, SMC are often left exposed. In a futile effort to repair the wound, the cells proliferate and clog the artery.

According to the present invention, there is now provided use of a compound of formula I:

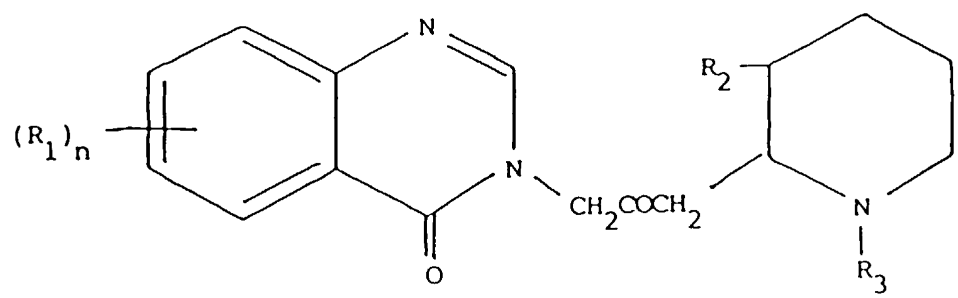

(I)

wherein: n = 1 or 2

$R_1$ is a member of the group consisting of hydrogen, halogen, nitro, benzo, lower alkyl, phenyl and lower alkoxy;
$R_2$ is a member of the group consisting of hydroxy, acetoxy, and lower alkoxy; and
$R_3$ is a member of the group consisting of hydrogen and lower alkenoxy-carbonyl

or a physiologically acceptable salt thereof for the manufacture of a pharmaceutical composition for preventing restenosis by the inhibition of vascular smooth cell proliferation, the composition comprising a pharmaceutically acceptable carrier and the compound or the physiologically acceptable salt thereof being an active ingredient of the composition.

Preferably the compound is halofuginone.

The carrier may be a liquid and the composition a solution.

The amount of halofuginone incorporated in the pharmaceutical composition may vary widely. Factors considered when determining the precise amount are well-known to those skilled in the art. Examples of such factors include, but are not limited to, the subject being treated, the specific pharmaceutical carrier, route of administration being employed, and the frequency with which the composition is to be administered.

As stated above, the compounds of formula I are administered in a pharmaceutical composition which comprises the compound and a pharmaceutically acceptable carrier. As used herein, the term "pharmaceutically acceptable carrier" encompasses any of the standard pharmaceutically accepted carriers, such as a phosphate-buffered saline solution, water, emulsions such as an oil/water emulsion or a triglyceride emulsion and various types of wetting agents. An example of an acceptable triglyceride emulsion useful in the intravenous and intraperitoneal administration of the compounds is the triglyceride emulsion commercially known as Intralipid®.

The administration of the pharmaceutical composition may be effected by any of the well-known methods, including, but not limited to, intravenous, intraperitoneal, intramuscular, or subcutaneous administration.

The invention will now be described in connection with certain preferred embodiments in the following examples and with reference to the appended figures, so that aspects thereof may be more fully understood and appreciated.

In the drawings:

| | |
|---|---|
| Fig. 1 | is a characteristic curve showing the inhibitory effect of halofuginone on SMC proliferation; |
| Fig. 2 | is a characteristic curve showing reversion of the antiproliferative effect of halofuginone on SMC; |
| Figs. 3a and 3b | respectively are a bar graph and a characteristic curve, showing the effect of halofuginone on $^3$H-thymidine incorporation into vascular SMCs; |
| Fig. 4 | is a characteristic curve showing the effect of halofuginone on vascular endothelial cell prolifer- |

| | |
|---|---|
| | ation; |
| Figs. 5a and 5b | respectively are a bar graph and a characteristic curve, showing the effect of halofuginone on $^3$H-thymidine incorporation into vascular endothelial cells; |
| Fig. 6 | is a bar graph showing antiproliferative effect of halofuginone on 3T3 fibroblasts; |
| Fig. 7 | is a bar graph showing the inhibitory effect of halofuginone on the mitogenic activity of bFGF; |
| Figs. 8a and 8b | are color light micrographs of the central artery of a rabbit ear after being subjected to crush injury, respectively showing an untreated artery and an artery treated according to the present invention; and |
| Fig. 9 | is a graph showing the effect of halofuginone on injury-induced artery stenosis. |

**EXAMPLES**

**1) Experimental Procedures**

**Cells**

SMC were isolated from the bovine aortic media as previously described [see, e.g., J.J. Castellot, et al., "Structural Determinants of the Capacity of Heparin to Inhibit the Proliferation of Vascular Smooth Muscle Cells: Evidence for a Pentasaccharide Sequence that Contains a 3-0-Sulfate Group," J. Cell Biol., Vol. 102, pp. 1979-1984 (1986); and A. Schmidt, et al., "The Antiproliferative Activity of Arterial Heparan Sulfate Resides in Domains Enriched with 2-0-Sulfated Uronic Acid Residues," J. Biol. Chem., Vol. 267, pp. 19242-19247 (1992)].

Briefly, the abdominal segment of the aorta was removed and the fascia cleaned away under a dissecting microscope. The aorta was cut longitudinally, and small pieces of the media were carefully stripped from the vessel wall. Two or three such strips, with average dimensions of 2-3 mm, were placed in 100 mm tissue culture dishes containing DMEM (4.5 g glucose/liter), supplemented with 10% FCS, 100 U/ml penicillin and 100 μg/ml streptomycin. Within 7-14 days, large patches of multilayered cells migrated from the explants. Approximately 1 week later, the cells were subcultured into 100-mm tissue culture plates (4-6x$10^5$ cells/plate). The cultures (passage 3-8) exhibited typical morphological characteristics of vascular SMC and the cells were specifically stained with monoclonal antibodies that selectively recognize the muscle form of actin (HF-35). This antibody does not recognize endothelial cells or fibroblasts.

Cultures of vascular endothelial cells were established from bovine aorta, as previously described by D. Gospodarowicz, et al. ["Clonal Growth of Bovine Endothelial Cells: Fibroblast Growth Factor as a Survival Agent," Proc. Natl. Acad. Sci. U.S.A., Vol. 73, p. 4120 (1979)]. Stock cultures were maintained in DMEM (1 g glucose/liter) supplemented with 10% calf serum, 50 U/ml penicillin, and 50 μg/ml streptomycin at 37°C in 10% $CO_2$ humidified incubators. Partially purified brain-derived bFGF (100 ng/ml) was added every other day during the phase of active cell growth [D. Gospodarowicz, et al., ibid., and I. Vlodavsky, et al., "Vascular Endothelial Cells Maintained in the Absence of Fibroblast Growth Factor Undergo Structural and Functional Alterations That Are Incompatible with Their In Vivo Differentiated Properties," J. Cell Biol., Vol 83, pp. 468-486 (1979)].

Cell Proliferation; $^3$H-Thymidine Incorporation

SMCs were plated (4x$10^4$ cells/16 mm well) in DMEM supplemented with 10% FCS. 24 hours after seeding, the medium was replaced with medium containing 0.2% FCS, and 48 hours later, the cells were exposed to growth stimulants and $^3$H-thymidine (1 μCi/well) for an additional 24-48 hours. DNA synthesis was assayed by measuring the radioactivity incorporated into trichloroacetic acid insoluble material [M. Benezra, et al., "Reversal of bFGF Autocrine Cell Transformation by Aromatic Anionic Compounds," Cancer Res., Vol. 52, pp. 5656-5662 (1992)].

**Growth Rate**

SMCs (1.5x$10^4$ cells/well) were seeded into 24 well culture plates and exposed to growth stimulants as described above. 1 to 6 days after seeding, the cells were fixed with 2.5% formaldehyde in PBS. The plates were immersed in a bath of 0.1 M borate buffer (pH 8.5), stained (1 h, 24°C) with methylene blue (1% in 0.1 M borate buffer, pH 8.5) and washed four times in water. This procedure removed practically all non-cell-bound dye. Specific cell incorporated methylene blue was dissolved withy 0.5 ml of 0.1 N HCl (1 h, 25°C) and determined by measuring the absorbency at 620 nm (Bar-Shavit, et al., ibid.). The initial cell plating density was chosen to ensure a linear relationship between cell number and absorbance at the end of the experiment. In each experiment, 3 wells were fixed before adding the test compound to determine the initial average absorbance. This value was used to calculate doubling times (DT) of control and drug-treated cells, using the following equation:

$$DT = \ln 2/\mathrm{IN}\ [(OD_t/OD_c)/h]$$

wherein:

DT = doubling time in hours;
$OD_t$ = optical density of a test well at the end of the experiment;
$OD_c$ = optical density of a control well at the beginning of the experiment;
h = duration of incubation in hours

The growth rate was calculated by dividing the doubling time of drug-treated cells by that of control cells [A. Horowitz, et al., "In Vitro Cytotoxicity of Liposome-Encapsulated Doxorubicin: Dependence on Liposome Composition and Drug Release," Biochim. Biophys. Acta, Vol. 1109, pp. 203-209 (1992)].

**Cell Number**

SMCs were seeded (2.5x103 cells/well) into 24-well plates in DMEM (4.5 g glucose/liter), supplemented with 10% FCS and allowed to attach for 6 hours [A. Schmidt, et al., "The Antiproliferative Activity of Arterial Heparan Sulfate Resides in Domains Enriched with 2-0-Sulfated Uronic Acid Residues," J. Biol. Chem., Vol. 267, pp. 19242-19247 (1992)]. The medium was removed and experimental medium (with or without halofuginone) containing 10% FCS was added to quadruplicate wells. After 4 days of incubation, the cell number was determined, using a Coulter counter (Schmidt, et al., ibid.). The degree of inhibition was calculated from the following formula:

% Inhibition = 1-net growth in presence of halofuginone/net growth in control x 100

The net growth was determined by subtracting the initial cell number from the final cell number.

**2) Experimental Results**

**i) Antiproliferative Effect of Halofuginone toward Vascular SMC**

**Growth Rate**

Sparsely seeded vascular SMC were exposed to 10% FCS in the absence and presence of increasing concentrations of halofuginone. The cells were dissociated with STV and counted daily. As shown in Fig. 1, 80-90% inhibition of SMC proliferation was obtained in the presence of 75 ng/ml halofuginone, with an almost complete inhibition at 125 ng/ml.

In another experiment, the SMCs were exposed to halofuginone for 48 hours, followed by removal of the drug and subsequent growth in regular growth medium. As demonstrated in Fig. 2, removal of the drug resulted in a gain of an accelerated growth rate similar to that of the untreated SMCs.

**$^3$H-Thymidine Incorporation**

Subconfluent vascular SMCs maintained in a medium containing 10% FCS were exposed (48 hours, 37°C) to $^3$H-thymidine in the absence and presence of increasing concentrations of halofuginone. As demonstrated in Fig. 3a, complete inhibition of DNA synthesis was observed at 0.15 μg/ml halofuginone, while 65% inhibition was obtained at a concentration as low as 0.05 μg/ml (Fig. 3b).

**ii) Antiproliferative Effect toward Vascular Endothelial Cells and 3T3 Fibroblasts**

**Vascular Endothelial Cells**

Sparsely seeded bovine aortic endothelial cells were cultured in medium containing 10% CS in the absence and presence of increasing concentrations of halofuginone. The cells were dissociated with 0.05% trypsin and 0.02% EDTA and counted daily. Inhibition of endothelial cell proliferation was observed primarily during the first 4 days, in cells treated with relatively high concentrations (0.1-0.125 μg/ml) of the drug (Fig. 4). Unlike the results with SMCs, the endothelial cells regained an almost normal growth rate (doubling time), starting on day 5 (Fig. 4), indicating that

vascular EC are less susceptible than vascular SMCs to the inhibitory effect of halofuginone. Thymidine incorporation studies revealed a 50% inhibition of DNA synthesis at 0.05 μg/ml halofuginone (Fig. 5).

**3T3 Fibroblasts**

Fig. 6 demonstrates that $^3$H-thymidine incorporation by actively growing 3T3 fibroblasts maintained in medium containing 10% FCS was almost completely inhibited in the presence of 0.025 μg/ml halofuginone, suggesting that fibroblasts are even more sensitive to the drug as compared to SMCs.

**Effect on bFGF-Induced Cell Proliferation**

Quiescent, growth arrested 3T3 fibroblasts maintained (48 hours) in medium containing 0.5% FCS are readily stimulated to proliferate by low concentrations at basic fibroblast growth factor (bFGF). Exposure to halofuginone (0.025 μg/ml) resulted in an almost complete inhibition of bFGF-stimulated thymidine incorporation in growth-arrested 3T3 fibroblasts (Fig. 7). This result suggests that halofuginone efficiently antagonizes the growth-promoting activity of bFGF.

**iii) Arterial Stenosis Caused by Physical Injury**

Adult New Zealand rabbits were anesthetized by intramuscular injection of ketamine (50 mg/kg). Physical injury was applied for 30 min. externally to the central artery of each ear [Banai, et al., Circulation Res., Vol. 69, pp. 748-756 (1992)]. After the operation, the rabbits were housed in accordance with Animal Welfare Act specifications. Halofuginone (0.2 ml of 0.09 mg/ml) was introduced subcutaneously around the physical crush area 1 hour after the crush and once every 24 hours during the first 4 days. On day 14, the animals were sacrificed and the ears fixed in 10% buffered formaldehyde for 72 hours. The crush sites were further trimmed at 1 mm intervals, dehydrated in ethanol and xylene, and embedded in paraffin. Serial (5μm) sections were stained by Movat pentachrome method. Computerized planimetry was performed at the site of the lesion and at an adjacent normal arterial segment displaced 2 mm from the location of the injury. Selection of the normal site was random; approximately one-half were proximal and one-half distal to the injury site. The lumen, the area cricumscribed by the internal elastic lamina ("original lumen") and the area circumscribed by the external border of the media (total vessel area) were traced, and the ratio between neointima and media was calculated. In all cases, the single section demonstrating the greatest extent of neointimal proliferation was selected for planimetry.

Referring now to Figs. 8a and 8b, there are seen light micrographs of the central artery of a rabbit ear 14 days after external crush injury (Movat staining of representative cross-sections).

In Fig. 8a, the SMCs are migrating from the media into the neointima through the disrupted internal elastic lamina and the artery lumen is narrowed by the protruding neointima in the untreated artery. As can be seen, there is striking neointimal formation and an almost complete obliteration of the arterial lumen.

In contradistinction, in Fig. 8b there is seen a rabbit ear artery subjected to crush injury and treatment with halofuginone. An almost complete inhibition of neo-intimal formation is observed.

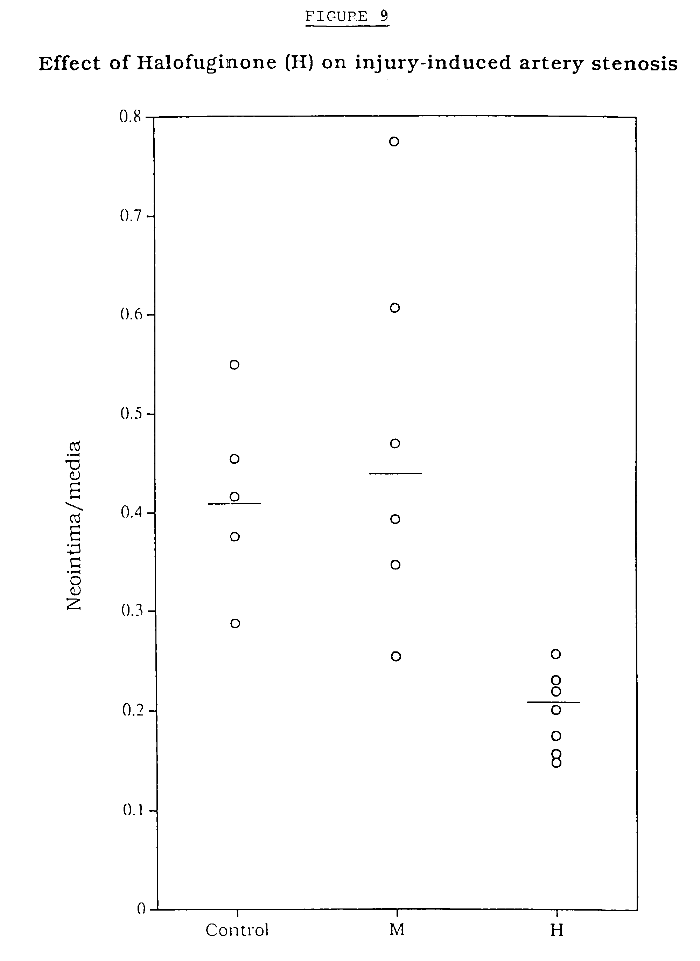

Fig. 9 shows a quantitative analysis of the ratio between enointima to media performed in control rabbits and rabbits treated with halofuginone (H) or a synthetic heparin-mimicking compound (M). Each point represents one rabbit.

**Conclusions**

Current approaches to inhibit the proliferation of vascular SMC utilize heparin, suramin, antibodies to various growth-promoting factors, anti-thrombin agents, and, most recently, antisense DNA technique. Heparin is a potent anticoagulant and its anti-proliferative activity is relatively small and subjected to major variations depending on the source and manufacturing company. Suramin is highly toxic at the effective dose, while antibodies are expensive, have a short half life and may elicit an immune response. Information on the antisense approach is new, and at present very limited.

The present invention, in its most preferred embodiment, utilizes a highly potent, inexpensive and non-toxic compound which inhibits the activity of various growth factors, including bFGF, and inhibits autocrine growth of vascular SMC and fibroblasts. Moreover, halofuginone is a low molecular weight compound which can be administered orally. The compound has been approved by the F.D.A. for use in farm animals. These characteristics make halofuginone a most promising clinically useful drug to inhibit restenosis.

Thus, the present invention provides for the use of halofuginone as a non-toxic compound that efficiently inhibits SMC proliferation, to provide an effective strategy for inhibiting the pathophysiology of arteriosclerosis, restenosis after coronary angioplasty, and neointimal proliferation in saphenous vein grafts.

**Claims**

1. Use of a compound of formula I:

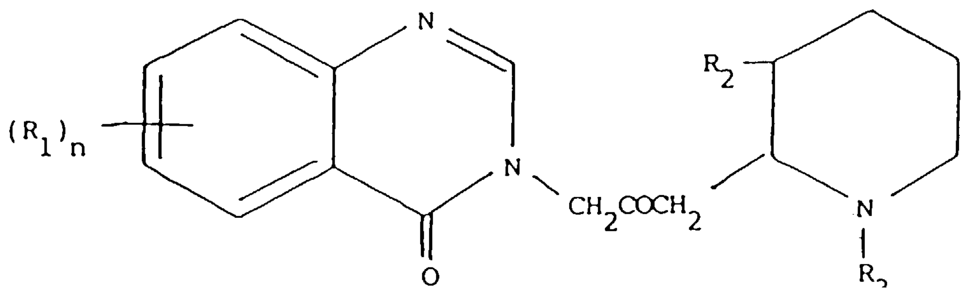

(I)

wherein: n = 1 or 2

$R_1$ is a member of the group consisting of hydrogen, halogen, nitro, benzo, lower alkyl, phenyl and lower alkoxy;
$R_2$ is a member of the group consisting of hydroxy, acetoxy, and lower alkoxy; and
$R_3$ is a member of the group consisting of hydrogen and lower alkenoxy-carbonyl

or a physiologically acceptable salt thereof for the manufacture of a pharmaceutical composition for preventing restenosis by the inhibition of vascular smooth cell proliferation, the composition comprising a pharmaceutically acceptable carrier and the compound or the physiologically acceptable salt thereof being an active ingredient of the composition.

2. A use according to claim 1, wherein the compound is halofuginone.

**Patentansprüche**

1. Verwendung einer Verbindung der Formel I:

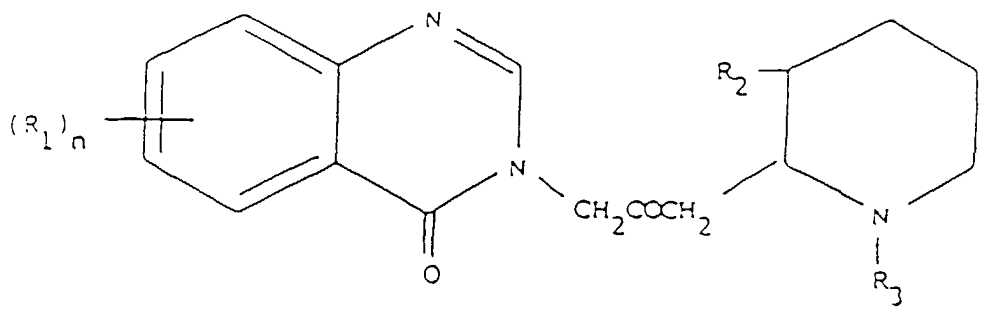

(I)

worin:

n = 1 oder 2

$R_1$ ist ein Bestandteil der Gruppe, bestehend aus Wasserstoff, Halogen, Nitro, Benzo, Niederalkyl, Phenyl und Niederalkoxy;

$R_2$ ist ein Bestandteil der Gruppe, bestehend aus Hydroxy, Acetoxy und Niederalkoxy; und

$R_3$ ist ein Bestandteil der Gruppe, bestehend aus Wasserstoff und Niederalkenoxy-carbonyl

oder eines physiologisch annehmbaren Salzes davon für die Herstellung einer pharmazeutischen Zusammensetzung zum Verhindern einer Restenose durch die Hemmung der Proliferation von vaskulären glatten Zellen, wobei die Zusammensetzung einen pharmazeutisch annehmbaren Träger und die Verbindung oder das physiologisch annehmbare Salz davon umfasst, welche(s) ein Wirkstoff der Zusammensetzung ist.

**2.** Verwendung nach Anspruch 1, wobei die Verbindung Halofuginon ist.

**Revendications**

**1.** Utilisation d'un composé de formule I :

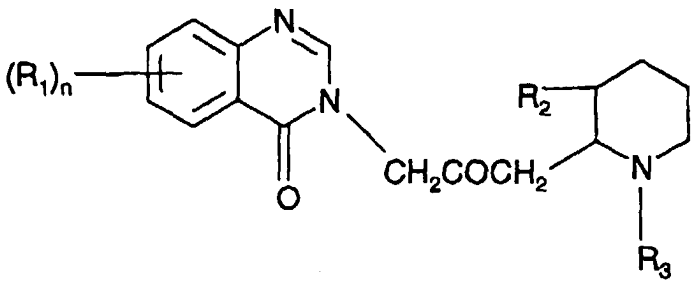

(I)

où :

n = 1 ou 2 ;
$R_1$ est un membre du groupe consistant en l'hydrogène, halogène, nitro, benzo, alkyle inférieur, phényle et alcoxy inférieur ;
$R_2$ est un membre du groupe consistant en hydroxyle, acétoxy et alcoxy inférieur ; et
$R_3$ est un membre du groupe consistant en l'hydrogène et alcénoxycarbonyle inférieur

ou d'un sel physiologiquement acceptable de celui-ci pour la fabrication d'une composition pharmaceutique pour prévenir une resténose par l'inhibition de la prolifération des cellules lisses vasculaires, la composition comprenant un support pharmaceutiquement acceptable et le composé ou son sel physiologiquement acceptable étant un ingrédient actif de la composition.

**2.** Utilisation selon la revendication 1, où le composé est l'halofuginone.

FIGURE 1

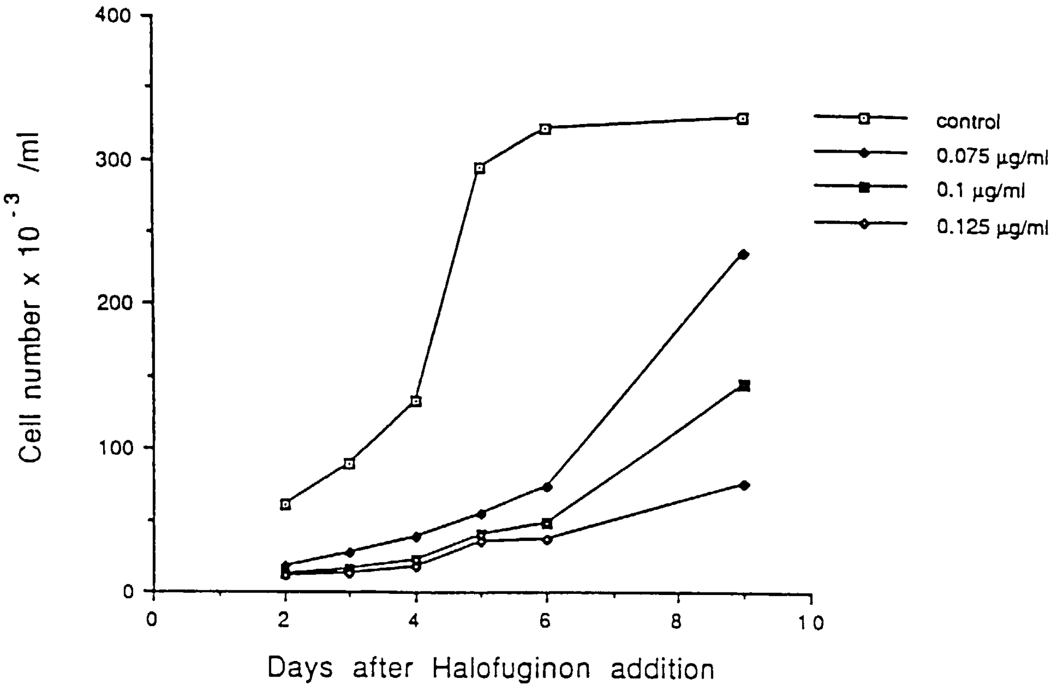


Reversion of the antiproliferative effect of Halofuginon on SMC

FIGURE 2

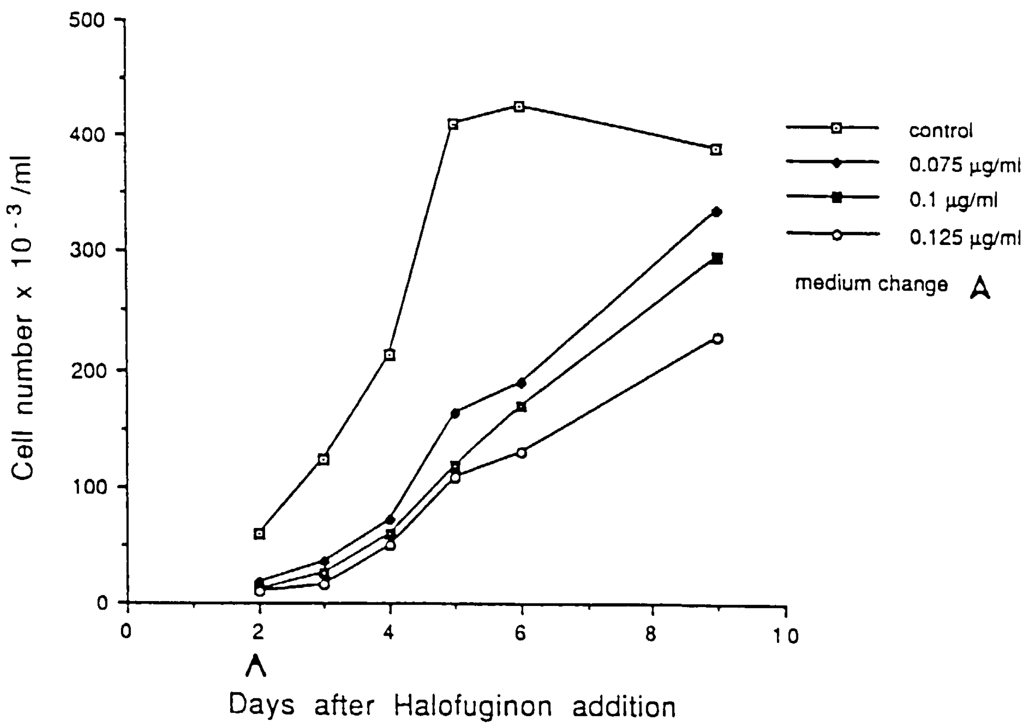

Effect of Halofuginon on $^3$H-Thymidine incorporation into vascular SMCs

a) $^3$H-thymidine incorporation
b) % inhibition

FIGURE 3a

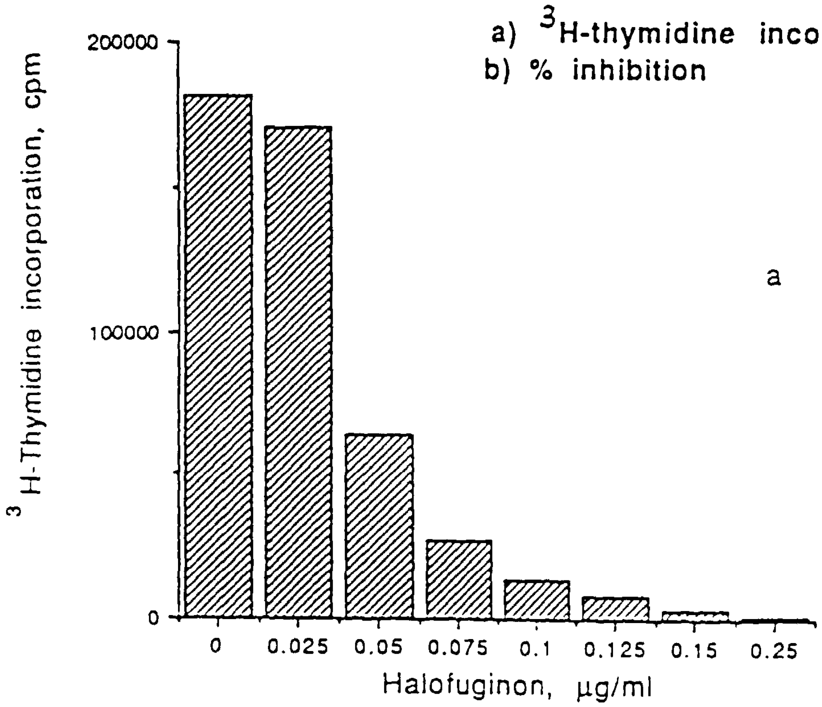


FIGURE 3b

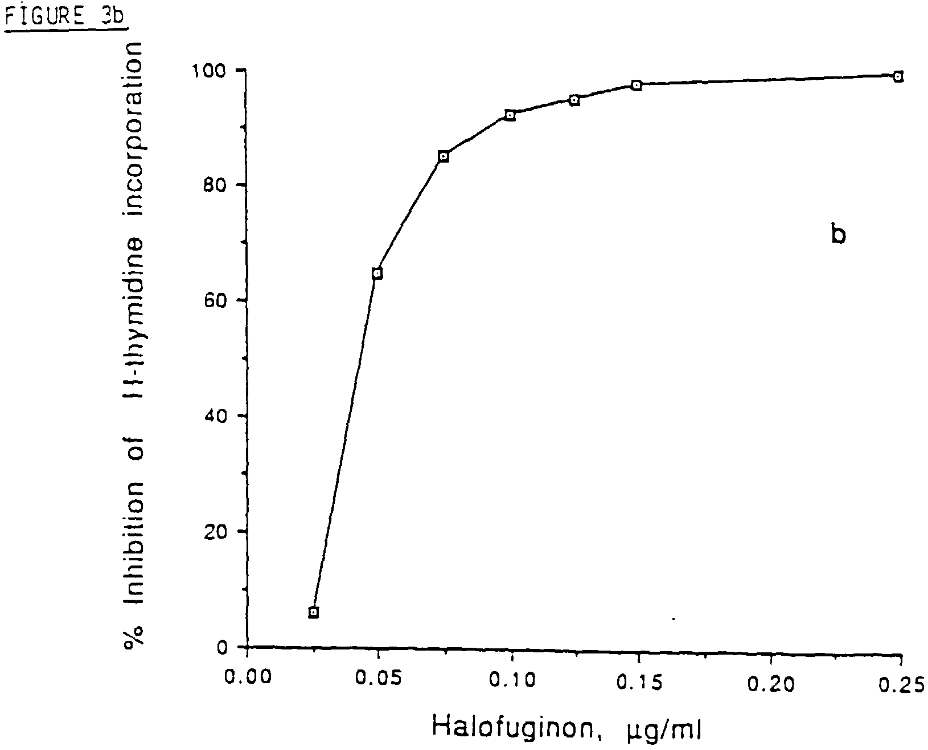

FIGURE 4

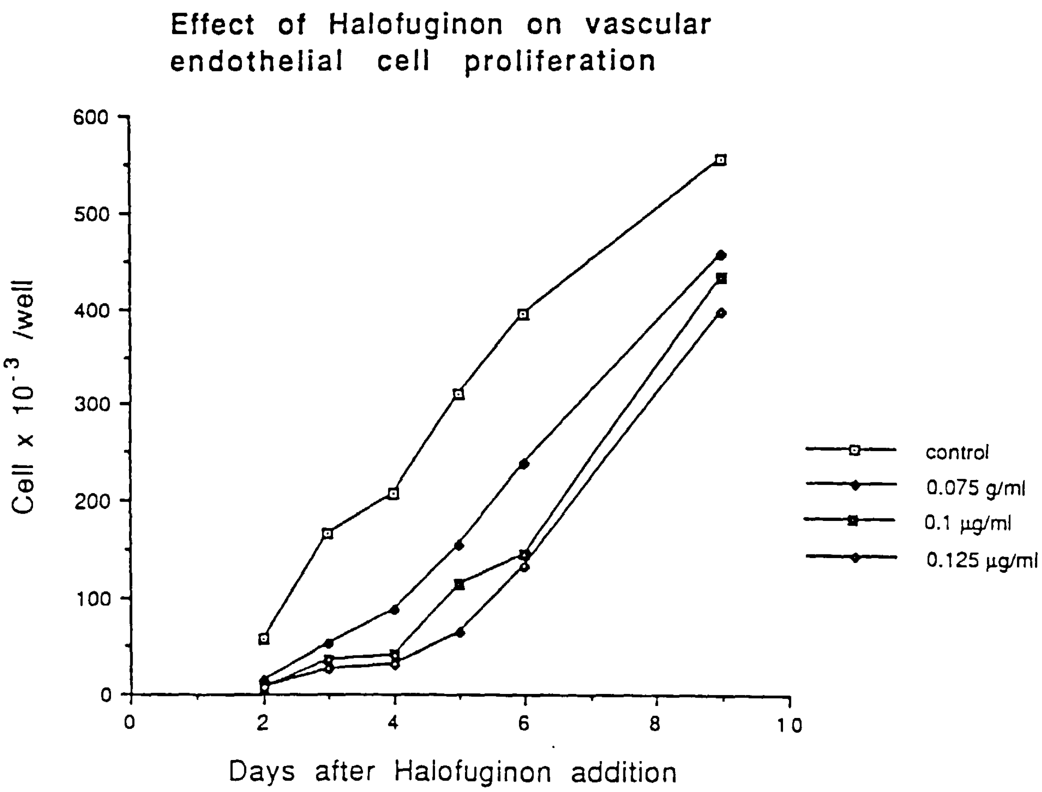
Effect of Halofuginon on vascular endothelial cell proliferation
600
500
400
300
200
100
0
Cell x 10^-3 /well
0
2
4
6
8
10
Days after Halofuginon addition
control
0.075 g/ml
0.1 µg/ml
0.125 µg/ml

Effect of Halofuginon on $^{3}$H-Thymidine incorporation into vascular endothelial cells
a) H-thymidine incorporation
b) % inhibition

FIGURE 5a

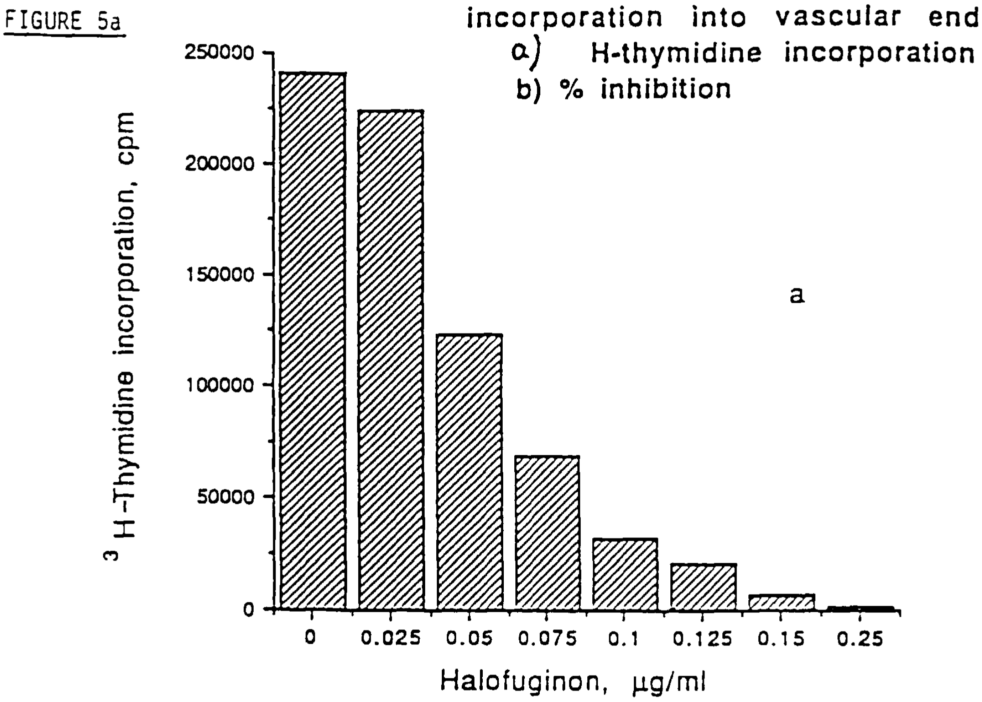


FIGURE 5b

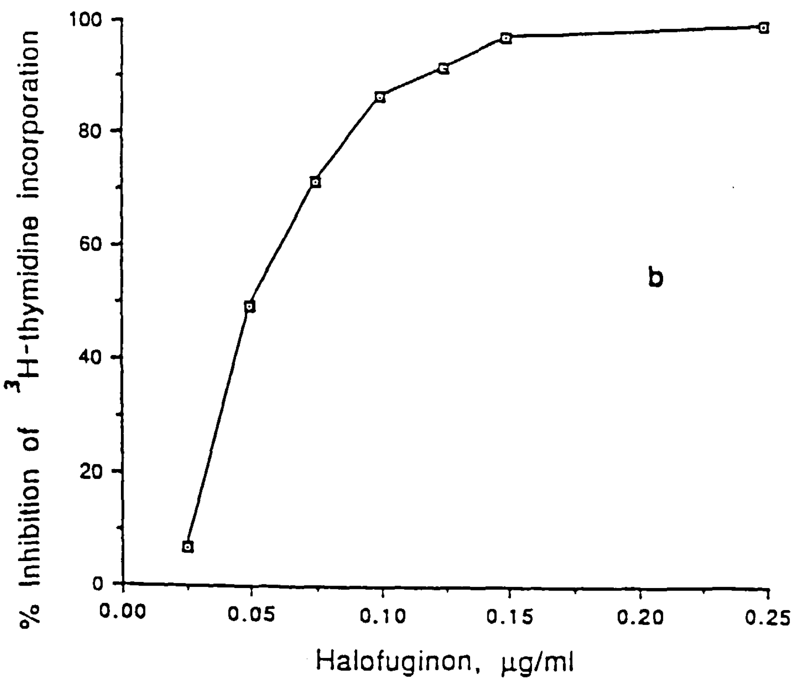

FIGURE 6

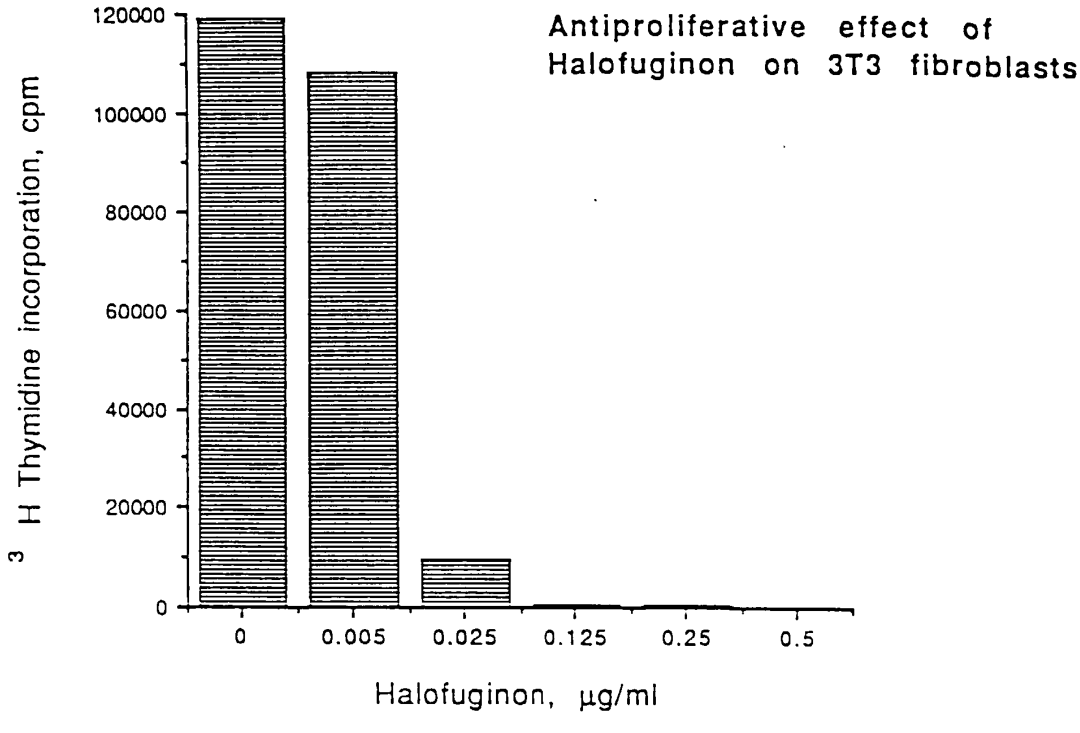
Antiproliferative effect of Halofuginon on 3T3 fibroblasts
3 H Thymidine incorporation, cpm
120000
100000
80000
60000
40000
20000
0
0
0.005
0.025
0.125
0.25
0.5
Halofuginon, µg/ml

FIGURE 7

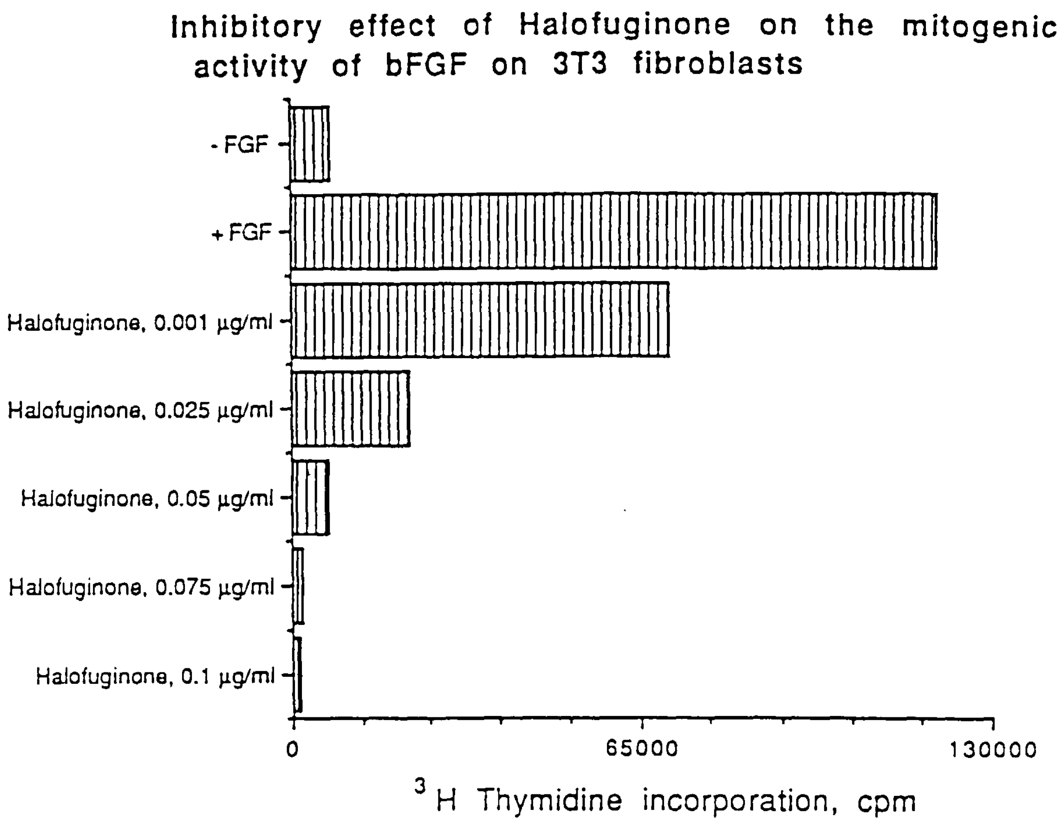
Inhibitory effect of Halofuginone on the mitogenic activity of bFGF on 3T3 fibroblasts
- FGF
+ FGF
Halofuginone, 0.001 μg/ml
Halofuginone, 0.025 μg/ml
Halofuginone, 0.05 μg/ml
Halofuginone, 0.075 μg/ml
Halofuginone, 0.1 μg/ml
0
65000
130000
3 H Thymidine incorporation, cpm

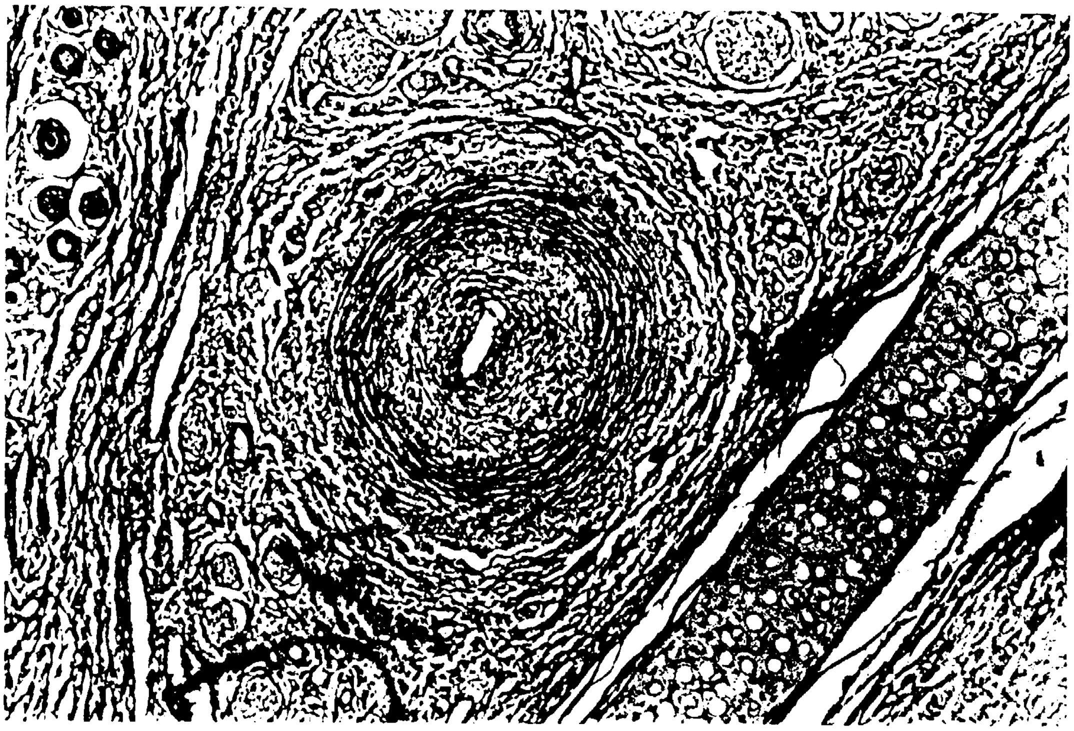

FIG. 8a

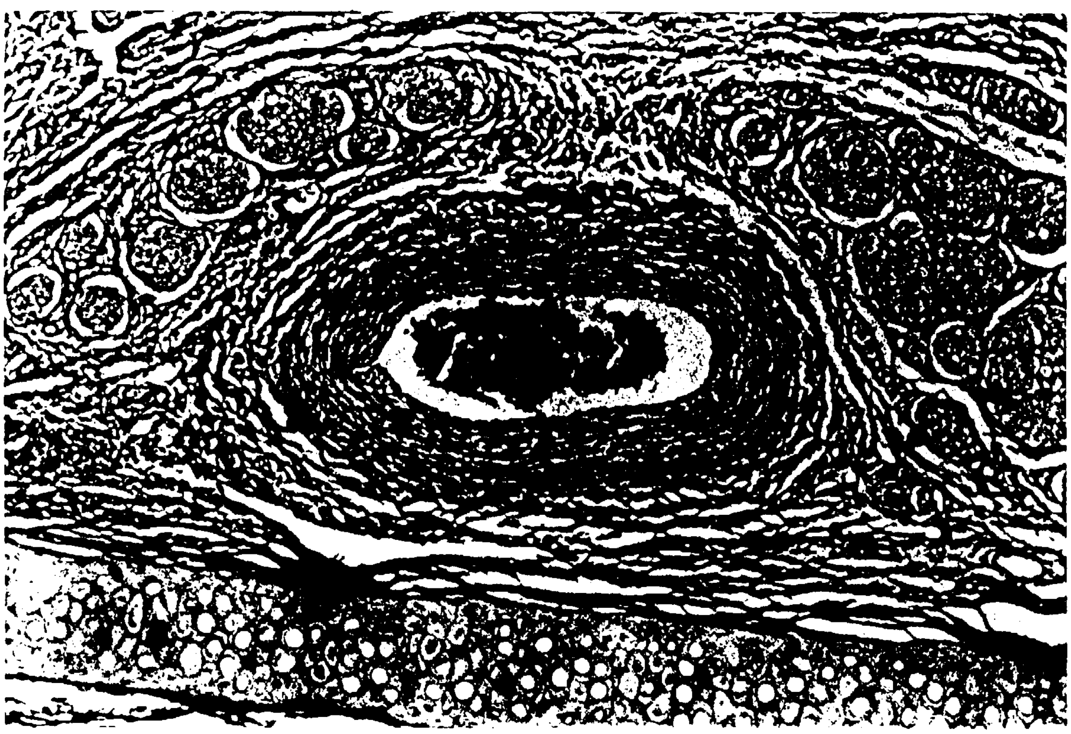

FIG. 8b

FIGURE 9

Effect of Halofuginone (H) on injury-induced artery stenosis